# EUROPÄISCHE PATENTSCHRIFT

(11) **EP 0 505 893 B1**
(45) Veröffentlichungstag und Bekanntmachung des Hinweises auf die Patenterteilung: **21.06.2000**
(21) Anmeldenummer: 92104571.2
(22) Anmeldetag: 17.03.1992
(51) Int. Cl.: C07D 471/04, A61K 31/435

(54) **Imidazopyridine**
Imidazopyridines
Imidazopyridines

(30) Priorität: 27.03.1991 DE 4110019
(43) Veröffentlichungstag der Anmeldung: 30.09.1992
(73) Patentinhaber: MERCK PATENT GmbH, 64271 Darmstadt (DE)
(72) Erfinder: Mederski, Werner, Dr., W-6106 Erzhausen (DE); Sombroek, Johannes, Dr., W-6100 Darmstadt (DE); Schelling, Pierre, Dr., W-6109 Mühltal (DE); Beier, Norbert, Dr., W-6107 Reinheim 5 (DE); Lues, Inge, Dr., W-6100 Darmstadt (DE); Minck, Klaus-Otto, Dr., W-6105 Ober-Ramstadt (DE)

(56) Entgegenhaltungen:
- EP-A- 0 399 731
- EP-A- 0 400 974
- EP-A- 0 407 342
- EP-A- 0 411 766
- EP-A- 0 415 886
- EP-A- 0 424 317
- EP-A- 0 434 038
- US-A- 4 880 804
- MATSUDA,A. ET AL.: "", J.MED.CHEM., Washington, , Band 34, Nr. , Seiten 2919 - 2922

## Beschreibung

Die Erfindung betrifft neue Imidazopyridinderivate der Formel I 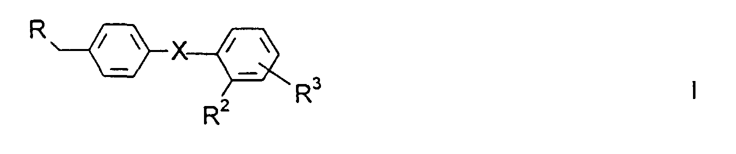 worin
R 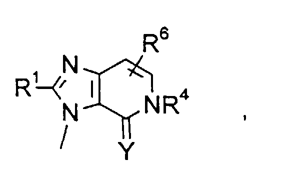
R¹ A mit bis zu 6 C-Atomen,
R² COOH, COOA, CN oder 5-Tetrazolyl,
R³ H,
R⁴ H, 5-Tetrazolyl-alkyl mit 1-6 C-Atomen mit "alkyl"-Teil, unsubstituiertes oder einfach durch Hal, COOH, COOA, CN, NO₂ oder 5-Tetrazolyl substituiertes Aralkyl mit 7-11 C-Atomen oder 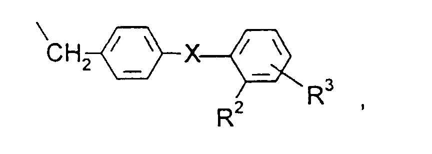
R⁶ H,
X fehlt,
Y O,
A Alkyl mit 1-6 C-Atomen und
Hal F, Cl, Br oder I bedeuten, sowie ihre Salze.

Aus EP 0 415 886 sind Azabenzimidazol-Verbindungen mit Angiotensin-II-antagonisierenden Eigenschaften bekannt. Andere Imidazo-verknüpfte 6-Ring-Heterocyclen kennt man aus EP 0 400 974. Angiotensin-II-Rezeptor blockierende Benzimidazole sind in US 4,880,804 beschrieben. Andere Imidazol-verknüpfte Heterocyclen zur Behandlung von Bluthochdruck und Herzkrankheiten kennt man aus EP 0 434 038. In EP 0 399 731 sind Azaindene, in EP 0 407 342 und EP 0 424 317 sind Pyrimidinderivate und in EP 0 411 766 sind Chinazolinone als AngiotensinII-Antagonisten genannt. Ferner sind in J. Med. Chem 34, 2919 (1991) andere heterocyclische Angiotensin-II-Antagonisten beschrieben.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie angiotensin II-antagonistische Eigenschaften und können daher zur Behandlung der angiotensin II-abhängigen Hypertension, des Aldosteronismus und der Herzinsuffizienz eingesetzt werden. Diese Wirkungen können nach üblichen in vitro- oder in vivo-Methoden ermittelt werden, wie sie z.B. in der US-PS 4 880 804 beschrieben sind, ferner von A.T. Chiu et al., J. Pharmacol. Exp. Therap. 250, 867-874 (1989), und von P.C. Wong et al., ibid 252, 719-725 (1990; in vivo, an Ratten).

Die Verbindungen der Formel I können als Arzneimittelwirkstoffe in der Human- und Veterinärmedizin eingesetzt werden, insbesondere zur Prophylaxe und/oder Therapie von Herz-, Kreislauf- und Gefäßkrankheiten, vor allem von Hypertonie, Herzinsuffizienz und Hyperaldosteronismus.

Gegenstand der Erfindung sind die Verbindungen der Formel I und ihre Satze sowie ein Verfahren zur Herstellung dieser Verbindungen sowie ihrer Salze, dadurch gekennzeichnet, daß man
(a) eine Verbindung der Formel II 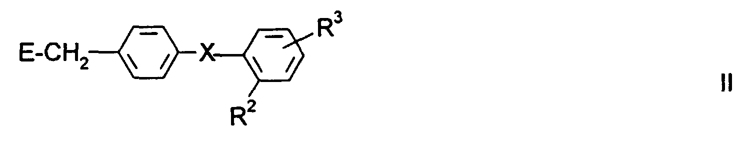 worin
   - E: Cl, Br, I oder eine freie oder reaktionsfähig funktionell abgewandelte OH-Gruppe bedeutet und
   - R², R³, und X: die in Anspruch 1 angegebene Bedeutung haben, mit einer Verbindung der Formel III

   H-R III

   worin
   - R: die in Anspruch 1 angegebene Bedeutung hat,
   oder
(b) zur Herstellung einer Verbindung der Formel I, worin R⁴ H und Y O bedeuten, eine Verbindung der Formel IV 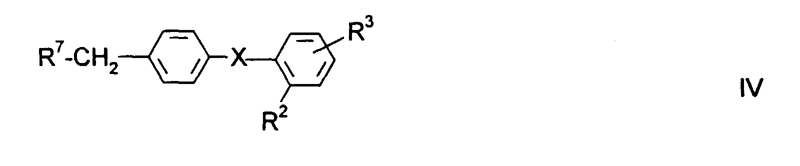 worin
   - R⁷: 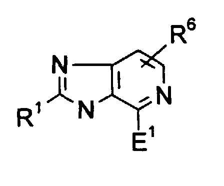 und
   - E¹: Cl, Br, I oder eine reaktionsfähig funktionell abgewandelte OH-Gruppe
   bedeuten und
   - R¹, R², R³, R⁶ und X: die in Anspruch 1 angegebenen Bedeutungen haben,
   mit einem solvolysierenden Mittel behandelt,
   oder
(c) eine Verbindung der Formel V 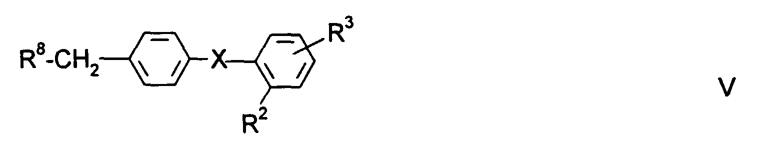 worin
   - R⁸: 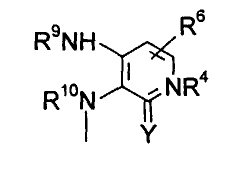
   - R⁹: R¹-CO oder H und
   - R¹⁰: H (falls R⁹ R¹-CO ist) oder R¹-CO (falls R⁹ H ist)
   bedeuten und
   - R¹, R², R³, R⁴, R⁶, X und Y: die in Anspruch 1 angegebenen Bedeutungen haben,
   mit einem cyclisierenden Mittel behandelt,
   oder
(d) eine Verbindung der Formel I aus einem ihrer funktionellen Derivate durch Behandeln mit einem solvolysierenden oder hydrogenolysierenden Mittel in Freiheit setzt,
und/oder daß man in einer Verbindung der Formel I einen oder mehrere Rest(e) R und/oder R² in einen oder mehrere andere Reste R und/oder R² umwandelt und/oder eine Base oder Säure der Formel I in eines ihrer Salze umwandelt.

Vor- und nachstehend haben die Reste bzw. Parameter R, R¹ bis R⁴, R⁶-R¹⁰, X, Y, A, Hal, E, E¹ die bei den Formeln I bis V angegebenen Bedeutungen, falls nicht ausdrücklich etwas anderes angegeben ist.

In den vorstehenden Formeln hat A 1-6, vorzugsweise 1, 2, 3 oder 4 C-Atome. A bedeutet vorzugsweise Methyl, weiterhin Ethyl, Propyl, Isopropyl, Butyl, Isobutyl, sek.-Butyl, oder tert.-Butyl, ferner auch Pentyl, 1-, 2-oder 3-Methylbutyl, 1,1-, 1,2- oder 2,2-Dimethylpropyl, 1-Ethylpropyl, Hexyl, 1-, 2-, 3-oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl. Alkenyl steht vorzugsweise für Vinyl, 1- oder 2-Propenyl, 1-Butenyl, ferner 1-Pentenyl oder 1-Hexenyl. Alkinyl steht vorzugsweise für Ethinyl, 1- oder 2-Propinyl, ferner 1-Butinyl, 1-Pentinyl oder 1-Hexinyl.

Hal bedeutet vorzugsweise F, Cl oder Br, aber auch J.

R ist ein von 3H-Imidazo[4,5-c]pyridin abgeleiteter Rest, genauer:

2-R¹-4-oxo-5-R⁴-6(oder 7)-R⁶-4,5-dihydro-3H-imidazo[4,5-c]pyridin-3-yl.

Der Rest R¹ ist vorzugsweise geradkettig und steht bevorzugt für A oder Alkenyl mit jeweils 3-6 C-Atomen, insbesondere Butyl, ferner Propyl, Pentyl, Hexyl, Allyl oder 1-Propenyl, ferner 1-Butenyl, 1-Pentenyl, 1-Hexenyl, 1-Propinyl, 1-Butinyl, 1-Pentinyl oder 1-Hexinyl.

Der Rest R² ist vorzugsweise CN, ferner bevorzugt 5-Tetrazolyl, COOH, COOCH₃ oder COOC₂H₅.

Der Rest R³ ist vorzugsweise H.

Der Rest R⁴ ist vorzugsweise H, ferner bevorzugt 5-Tetrazolyl-alkyl [insbesondere 5-Tetrazolyl-methyl, 2-(5-Tetrazolyl)-ethyl, 3-(5-Tetrazolyl)-propyl], wobei alle diese Reste jeweils insgesamt bis zu 6 C-Atome enthalten können. Außerdem ist der Rest R⁴ vorzugsweise unsubstituiertes oder einfach (vorzugsweise in o-Stellung) oder zweifach (vorzugsweise in 2,6-Stellung) substituiertes Aralkyl mit 7-11 C-Atomen, insbesondere Benzyl, 1- oder 2-Phenylethyl, 1-, 2- oder 3-Phenylpropyl, 1-, 2-, 3- oder 4-Phenylbutyl, o-, m- oder p-Fluorbenzyl, (bevorzugt) o-, m- oder p-Chlorbenzyl, o-, m- oder p-Brombenzyl, o-, m-, oder p-Methylbenzyl, o-, m- oder p-Trifluormethylbenzyl, o-, m- oder p-Methoxycarbonylbenzyl, o-, m- oder p-Ethoxycarbonylbenzyl, (bevorzugt) o-, m- oder p-Cyanbenzyl, o-, m- oder p-Carboxybenzyl, o-, m- oder p-Nitrobenzyl. Weiterhin kann der Rest R⁴ bevorzugt 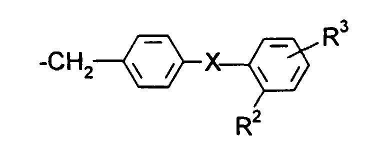 bedeuten.

Der Rest R⁶ ist vorzugsweise H.

Der Rest X fehlt vorzugsweise.

Der Rest Y ist vorzugsweise O.

Die Verbindungen der Formel I können ein oder mehrere chirale Zentren besitzen und daher in verschiedenen - optisch-aktiven oder optischinaktiven - Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat.

Vor allem bevorzugt sind Verbindungen der Formeln I, worin zusätzlich R³, R⁴ und/oder R⁶ H und/oder Y O bedeuten.

Unter diesen sind diejenigen Verbindungen bevorzugt, worin R² CN, COOH, COOCH₃, COOC₂H₅ oder 5-Tetrazolyl bedeutet.

Eine ganz besonders bevorzugte Gruppe von Verbindungen entspricht der Formel 1, worin
- R: 2-A-4,5-dihydro-4-oxo-5-R⁴-3H-imidazo[4,5-c]pyridin-3-yl,
- R²: COOH, COOCH3, CN oder 5-Tetrazolyl,
- R³: H,
- R⁴: H oder 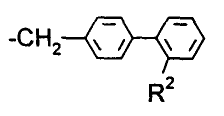 bedeuten und
- X: fehlt.

Eine kleine ausgewählte Gruppe bevorzugter Verbindungen entspricht der Formel I, worin
- R: einen 2-Butyl-4,5-dihydro-4-oxo-3H-imidazo[4,5-c]pyridin-3-yl-rest,
- R²: COOH, COOCH₃, CN oder 5-Tetrazolyl
- R³: H und
- Y: O bedeuten und
- X: fehlt.

Eine weitere ausgewählte Gruppe bevorzugter Verbindungen entspricht der Formel I, worin R⁴ 5-Tetrazolylalkyl mit jeweils 1-6 C-Atomen im Alkylteil, unsubstituiertes oder einfach durch Hal, COOH, COOA, CN, NO₂ oder 5-Tetrazolyl substituiertes Aralkyl mit 7-11 C-Atomen bedeutet.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; insbesondere aber US-PS 4 880 804 beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können vorzugsweise erhalten werden, indem man Verbindungen der Formel II mit Verbindungen der Formel III umsetzt. Besonders die Biphenylderivate der Formel I (worin X fehlt) sind auf diesem Wege gut erhältlich.

In den Verbindungen der Formel II bedeutet E vorzugsweise Cl, Br, I oder eine reaktionsfähig funktionell abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy).

Die Umsetzung von II mit III erfolgt zweckmäßig, indem man zunächst III durch Behandeln mit einer Base in ein Salz umwandelt, z.B. mit einem Alkalimetallalkoholat wie CH₃Ona in einem Alkohol wie CH₃OH oder mit einem Alkalimetallhydrid wie NaH in Dimethylformamid (DMF), und dieses dann in einem inerten Lösungsmittel, z.B. einem Amid wie DMF oder Dimethylacetamid oder einem Sulfoxid wie Dimethylsulfoxid (DMSO), mit II umsetzt, zweckmäßig bei Temperaturen zwischen -20 und 100°, vorzugsweise, zwischen 10 und 30°. Als Basen eignen sich auch Alkalimetallcarbonate wie Na₂CO₃ oder K₂CO₃ oder Alkalimetall-hydrogencarbonate wie NaHCO₃ oder KHCO₃.

Bei der Reaktion von II mit III kann man zwei, im Falle von III, R⁴ = H drei regioisomere Monosubstitutionsprodukte erhalten, nämlich die entsprechenden 1H- und 3H-Imidazo[4,5-c]pyridine, bei denen der neu eingeführte Substituent in 1-, 3- oder 5-Stellung steht. Außerdem sind Disubstitutionsprodukte erhältlich mit Substitution in 1- und 3- bzw. in 1- und 5- bzw. in 3- und 5-Stellung. Die Art und das Mengenverhältnis der Produkte der Formel I sind weitgehend vom Mengenverhältnis der Reaktionspartner II und III sowie den Reaktionsbedingungen abhängig. So kann man bei der Umsetzung äquimolarer Mengen von 4'-Brommethyl-biphenyl-2-carbonsäuremethylester ("IIa") und 2-Butyl-4-oxo-4,5-dihydro-1 (oder 3)H-imidazo[4,5-c]pyridin ("IIIa") in Gegenwart von CH₃ONa in Methanol die in 1- und 5- sowie in 3- und 5-Stellung disubstituierten Produkte isolieren; bei der Umsetzung von 4'-Brommethyl-2-cyanbiphenyl ("IIb") mit IIIa in Gegenwart von K₂CO₃ in DMF erhält man dagegen ganz überwiegend das in 3-Stellung monosubstituierte Produkt.

Die Verbindungen der Formel I sind ferner durch Solvolyse, insbesondere saure oder alkalische Hydrolyse, von Verbindungen der Formel IV erhältlich. In IV bedeutet der Rest E¹ vorzugsweise Cl. Eine Solvolyse von IV gelingt besonders vorteilhaft aber auch mit Silberacetat in Essigsäure bei Temperaturen zwischen 20° und Siedetemperatur.

Die Verbindungen der Formel I sind weiterhin durch Cyclisierung von Verbindungen der Formel V erhältlich. Diese Cyclisierung gelingt zweckmäßig durch Erhitzen mit Polyphosphorsäure, Essigsäure oder Diglyme auf Temperaturen zwischen etwa 80 und 180°, vorzugsweise zwischen 120 und 160°.

Man kann ferner eine Verbindung der Formel I aus einem ihrer funktionellen Derivate durch Behandeln mit einem solvolysierenden (z.B. hydrolysierenden) oder hydrogenolysierenden Mittel in Freiheit setzen.

So ist es möglich, nach einer der angegebenen Methoden eine Verbindung herzustellen, die der Formel I entspricht, aber an Stelle einer 5-Tetrazolylgruppe eine in 1- oder 2-Stellung funktionell abgewandelte (durch Schutzgruppe geschützte) 5-Tetrazolylgruppe enthält. Als Schutzgruppen eignen sich beispielsweise: Triphenylmethyl, abspaltbar mit HCl oder Ameisensäure in einem inerten Lösungsmittel oder Lösungsmittelgemisch, z.B. Ether/Dichlormethan/Methanol; 2-Cyanethyl, abspaltbar mit NaOH in Wasser/THF; p-Nitrobenzyl, abspaltbar mit H₂/Raney-Nickel in Ethanol.

Die Ausgangsstoffe, insbesondere diejenigen der Formel II sind teilweise bekannt. Falls sie nicht bekannt sind, können sie nach bekannten Methoden in Analogie zu bekannten Stoffen hergestellt werden. Verbindungen der Formel III sind neu. Solche der Formel III (R⁴ = H, Y = O) sind z.B. erhältlich durch Reaktion von Carbonsäuren der Formel R¹-COOH mit Verbindungen der Formel X 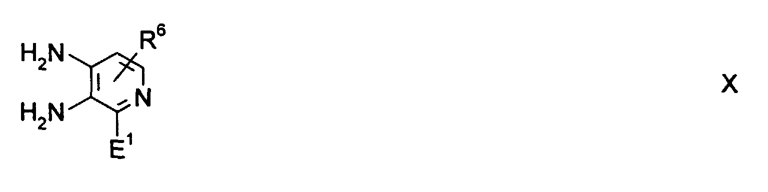 in Gegenwart von Polyphosphorsäure; dabei wird die Gruppe E¹ (vorzugsweise Cl) hydrolysiert.

Verbindungen der Formel IV sind beispielsweise erhältlich durch Umsetzung von Verbindungen der Formel H-R⁷ mit Verbindungen der Formel II unter den oben für die Reaktion von II mit III angegebenen Bedingungen.

Es ist ferner möglich, eine Verbindung der Formel I in eine andere Verbindung der Formel I umzuwandeln, indem man einen oder mehrere der Reste R und/oder R² in andere Reste R und/oder R² umwandelt, z.B. indem man Halogenatome (z.B. durch Reaktion mit Kupfer(I)cyanid) durch CN-Gruppen ersetzt und/oder Nitrilgruppen zu COOH-Gruppen hydrolysiert oder mit Derivaten der Stickstoffwasserstoffsäure, z.B. Natriumazid in N-Methylpyrrolidon oder Trimethylzinnazid in Toluol, zu Tetrazolylgruppen umsetzt.

So kann man beispielsweise freie Aminogruppen in üblicher Weise mit einem Säurechlorid oder -anhydrid acylieren oder mit einem unsubstituierten oder substituierten Alkyl-, Alkenyl-, Alkinyl- oder Aralkylhalogenid alkylieren, zweckmäßig in einem inerten Lösungsmittel wie Dichlormethan oder THF und/oder in Gegenwart einer Base wie Triethylamin oder Pyridin bei Temperaturen zwischen -60 und +30°. Von Bedeutung ist insbesondere die entsprechende Umwandlung eines Restes R, worin R⁴ = H ist, in einen anderen Rest R, worin R⁴ von H verschieden ist. Hier arbeitet man bevorzugt mit einem Säureamid wie DMF, N-Methylpyrrolidon, 1,3-Dimethyl-2-oxo-hexahydropyridin oder Phosphorsäure-hexamethyl-triamid, einem Alkohol wie Methanol oder tert.-Butanol, einem Ether wie THF oder einem halogenierten Kohlenwasserstoff wie Dichlormethan oder Gemischen davon als Lösungsmittel und/oder in Gegenwart eines Alkalimetallalkoholats wie Natriummethylat oder Kalium-tert.-butylat, eines Alkalimetallhydrids wie Natrium- oder Kaliumhydrid, eines Alkalimetallcarbonats wie Natrium- oder Kaliumcarbonat, eines Alkalilmetallbicarbonats wie Natrium- oder Kaliumbicarbonat oder eines tertiären Amins wie Triethylamin oder Ethyldilsopropylamin bei Temperaturen zwischen -30 und 200, vorzugsweise zwischen 20 und 60°.

Umsetzung von Nitrilen der Formel I (R² = CN) mit Derivaten der Stickstoffwasserstoffsäure führt zu Tetrazolen der Formel I (R² = 5-Tetrazolyl). Bevorzugt verwendet man Trialkylzinnazide wie Trimethylzinnazid in einem inerten Lösungsmittel, z.B. einem aromatischen Kohlenwasserstoff wie Toluol bei Temperaturen zwischen 20 und 150°, vorzugsweise zwischen 80 und 140°, oder Natriumazid in N-Methylpyrrolidon bei Temperaturen zwischen etwa 100 und 200°.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern, so können anorganische Säuren verwendet werden, z.B. Schwefelsäure, Salpetersäure, Halogenwasserstoffsäuren wie Chlorwasserstoffsäure oder Bromwasserstoffsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Andererseits können Verbindungen der Formel I, die COOH- oder Tetrazolylgruppen enthalten, mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden. Die Kaliumsalze der Tetrazolylderivate sind besonders bevorzugt.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässrige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z.B. Fluorchlorkohlenwasserstoffe) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine, Diuretika, Antiphlogistika.

Die erfindungsgemäßen Substanzen werden in der Regel in Analogie zu anderen bekannten, im Handel befindlichen Präparaten, insbesondere aber in Analogie zu den in der US-PS 4 880 804 beschriebenen Verbindungen verabreicht, vorzugsweise in Dosierungen zwischen etwa 1 mg und 1 g, insbesondere zwischen 50 und 500 mg pro Dosierungseinheit. Die tägliche Dosierung liegt vorzugsweise zwischen etwa 0,1 und 500 mg/kg, insbesondere 1 und 100 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkomibination und Schwere der jeweiligen Erkrankung, welcher die Therapie gilt. Die orale Applikation ist bevorzugt.

Vor- und nachstehend sind alte Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation.

Rf = Rf-Wert, bestimmt dünnschichtchromatographisch an Kieselgel; Laufmittel: Ethylacetat, wenn nicht anders angegeben.

### Beispiel 1

Eine Lösung von 0,4 g Na in 20 ml Methanol wird innerhalb 15 Min. zugetropft zu einer Lösung von 3,2 g 2-Butyl-4-oxo-4,5-dihydro-1(oder 3)H-imidazo[4,5-c]pyridin ("IIIa") in 75 ml Methanol. Man rührt noch 30 Min. bei 20°, dampft ein, löst den Rückstand in 20 ml DMF und tropft bei 0° unter Rühren eine Lösung von 5,2 g 4'-Brommethyl-biphenyl-2-carbonsäure-methylester (IIa) in 10 ml DMF hinzu. Man rührt 16 Std. Bei 20°, dampft ein, arbeitet wie üblich auf chromatographiert an Kieselgel und erhält mit Methyl-tert.-butylether/Methanol (9,5 : 0,5 bis 9:1) nacheinander:
2-Butyl-3,5-bis-(2'-methoxycarbonyl-biphenylyl-4-methyl)-4,5-dihydro-4-oxo-3H-imidazo[4,5-c]pyridin, Öl;
2-Butyl-1-(2'-methoxycarbonyl-biphenylyl-4-methyl)-4,5-dihydro-4-oxo-1H-imidazo[4,5-c]pyridin, F. 224°;
2-Butyl-5-(2'-methoxycarbonyl-biphenyl-4-methyl)-4,5-dihydro-4-oxo-1(oder 3)H-imidazo[4,5-c]pyridin, F. 151°;
2-Butyl-3-(2'-methoxycarbonyl-biphenylyl-4-methyl)-4,5-dihydro-4-oxo-3H-imidazo[4,5-c]pyridin, F. 186°;
2-Butyl-1,5-bis-(2'-methoxycarbonyl-biphenyl-4-methyl)-4,5-dihydro-4-oxo-1H-imidazo[4,5-c]pyridin, F. 68°.

### Herstellung des Ausgangsmaterials IIIa:

Ein Gemisch von 16,2 g 3,4-Diamino-2-chlorpyridin, 14 ml Valeriansäure und 300 g Polyphosphorsäure wird unter Rühren 8 Std. auf 100-140°, dann 5 Std. auf 170-180° erhitzt. Man kühlt ab, gießt auf Eis und gibt Natronlauge bis pH 9 hinzu. Nach Einengen und üblicher Aufarbeitung erhält man IIIa, F. 285-290°.

Analog erhält man aus IIa und 2-Butyl-6-chlor-4-oxo-4,5-dihydro-1(oder 3)H-imidazo[4,5-c]pyridin (F. 235-240°; erhältlich aus 3,4-Diamino-2,6-dichlorpyridin und Valeriansäure):
2-Butyl-6-chlor-3,5-bis-(2'-methoxycarbonyl-biphenyl-4-methyl)-4,5-dihydro-4-oxo-3H-imidazo[4,5-c]pyridin
2-Butyl-6-chlor-1-(2'-methoxycarbonyl-biphenylyl-4-methyl)-4,5-dihydro-4-oxo-1(oder 3)H-imidazo[4,5-c]pyridin
2-Butyl-6-chlor-5-(2'-methoxycarbonyl-biphenylyl-4-methyl)-4,5-dihydro-4-oxo-1(oder 3)H-imidazo[4,5-c]pyridin
2-Butyl-6-chlor-3-(2'-methoxycarbonyl-biphenyl-4-methyl)-4,5-dihydro-4-oxo-3H-imidazo[4,5-c]pyridin
2-Butyl-6-chlor-1,5-bis-(2'-methoxycarbonyl-biphenylyl-4-methyl)-4,5-dihydro-4-oxo-1H-imidazo[4,5-c]pyridin.

### Beispiel 2

Ein Gemisch von 0,7 g IIIa, 0,5 g K₂CO₃ und 40 ml DMF wird 10 Min. bei 20° gerührt. Man tropft unter Rühren innerhalb 45 Min. eine Lösung von 1 g 4'-Brommethyl-2-cyan-biphenyl in 5 ml DMF hinzu, rührt noch 5 Std. bei 20°, dampft ein und arbeitet wie üblich auf. Man erhält nach Chromatographie an Kieselgel (Dichlormethan/Methanol 98:2 bis 9:1):
2-Butyl-1-(2'-cyan-biphenylyl-4-methyl)-4,5-dihydro-4-oxo-1H-imidazo[4,5-c]pyridin,
2-Butyl-3-(2'-cyan-biphenylyl-4-methyl)-4,5-dihydro-4-oxo-3H-imidazo[4,5-c]pyridin, F. 179° (Hauptprodukt),
2-Butyl-5-(2'-cyan-biphenylyl-4-methyl)-4,5-dihydro-4-oxo-1(oder 3)H-imidazo[4,5-c]pyridin,
2-Butyl-1,5-bis-(2'-cyan-biphenylyl-4-methyl)-4,5-dihydro-4-oxo-1H-imidazo[4,5-c]pyridin,
2-Butyl-3,5-bis-(2'-cyan -biphenylyl-4-methyl)-4,5-dihydro-4-oxo-3H-imidazo[4,5-c]pyridin, F. 83°.

Analog erhält man mit 2'-Nitro-biphenylyl-4-methylbromid:
2-Butyl-1-(2'-nitro-biphenylyl-4-methyl)-4,5-dihydro-4-oxo-1H-imidazo[4,5-c]pyridin
2-Butyl-3-(2'-nitro-biphenylyl-4-methyl)-4,5-dihydro-4-oxo-3H-imidazo[4,5-c]pyridin
2-Butyl-5-(2'-nitro-biphenylyl-4-methyl)-4,5-dihydro-4-oxo-1(oder 3)H-imidazo[4,5-c]pyridin
2-Butyl-1,5-bis-(2'-nitro-biphenylyl-4-methyl)-4,5-dihydro-4-oxo-1H-imidazo[4,5-c]pyridin
2-Butyl-3,5-bis-(2'-nitro-biphenylyl-4-methyl)-4,5-dihydro-4-oxo-3H-imidazo[4,5-c]pyridin.

### Beispiel 3

Ein Gemisch von 4 g 2-Butyl-3-(2'-cyan-biphenylyl-4-methyl)-4-chlor-3H-imidazo[4,5-c]pyridin [erhältlich durch Kondensation von 3,4-Diamino-2-chlorpyridin mit Valeriansäure analog Beispiel 4 zu 2-Butyl-4-chlor-1(oder 3)H-imidazo[4,5-c]pyridin (F. 65°) und Reaktion mit IIIa analog Beispiel 2], 2 g CH₃COOAg und 40 ml Essigsäure wird 16 Std. gekocht. Man filtriert, dampft ein, arbeitet den Rückstand wie üblich auf und erhält 2-Butyl-3-(2'-cyan-biphenylyl-4-methyl)-4,5-dihydro-4-oxo-3H-imidazo[4,5-c]pyridin, F. 179°.

### Beispiel 4

Ein Gemisch von 1,02 g Valeriansäure, 3,59 g 4-Amino-2-oxo-3-[2'-(5-tetrazolyl)-biphenylyl-4-methyl-amino]-1,2-dihydropyridin [erhältlich durch Reaktion von 3-Amino-4-benzylamino-1,2-dihydro-2-oxo-pyridin mit 4-Brommethyl-2'-cyan-biphenyl zu 4-Benzylamino-3-(2'-cyan-biphenylyl-4-methyl-amino)-1,2-dihydro-2-oxo-pyridin, Reaktion mit Trimethylzinnazid gemäß Beispiel 13 zu 4-Benzylamino-3-[2'-(5-tetrazolyl)-biphenylyl-4-methyl-amino]-1,2-dihydro-2-oxo-pyridin und hydrogenolytische Entfernung der Benzylgruppe] und 50 g Polyphosphorsäure wird 5 Std. auf 140° erhitzt. Als Zwischenprodukte entstehen in situ 4-Amino-2-oxo-3-[N-2'-(5-tetrazolyl)-biphenylyl-4-methyl-N-valeryl-amino]-1,2-dihydro-pyridin und 2-Oxo-3-[2'-(5-tetrazolyl)-biphenylyl-4-methyl-amino]-4-valerylamino-1,2-dihydro-pyridin. Man kühlt ab, gießt auf Eis, macht mit Natronlauge alkalisch, arbeitet wie üblich auf und erhält 2-Butyl-4,5-dihydro-4-oxo-3-[2'-(5-tetrazolyl)-biphenylyl-4-methyl]-3H-imidazo[4,5-c]pyridin, F. 167°.

### Beispiel 5

Ein Gemisch von 1 g 2-Butyl-1-(2'-methoxycarbonyl-biphenylyl-4-methyl)-4,5-dihydro-4-oxo-1H-imidazo[4,5-c]pyridin, 12 ml 2 n wässerige NaOH-Lösung und 43 ml Ethanol wird 2 Std. gekocht, dann eingedampft. Nach Ansäuern mit HCl auf pH 3 erhält man 2-Butyl-1-(2'-carboxy-biphenylyl-4-methyl)-4,5-dihydro-4-oxo-1H-imidazo[4,5-c]pyridin, das abfiltriert, mit Wasser gewaschen und getrocknet wird, F. 286°.

Analog erhält man durch Verseifung der entsprechenden Methylester:
2-Butyl-3-(2'-carboxy-biphenylyl-4-methyl)-4,5-dihydro-4-oxo-3H-imidazo[4,5-c]pyridin, F. 275°
2-Butyl-5-(2'-carboxy-biphenylyl-4-methyl)-4,5-dihydro-4-oxo-1(oder 3)H-imidazo[4,5-c]pyridin
2-Butyl-1,5-bis-(2'-carboxy-biphenylyl-4-methyl)-4,5-dihydro-4-oxo-1H-imidazo[4,5-c]pyridin, Dihydrat, F. 137°
2-Butyl-3,5-bis-(2'-carboxy-biphenylyl-4-methyl)-4,5-dihydro-4-oxo-3H-imidazo[4,5-c]pyridin, F. 165°.

### Beispiel 6

Eine Lösung von 3,82 g 2-Butyl-3-(2'-cyan-biphenylyl-4-methyl)-4,5-dihydro-4-oxo-3H-imidazo[4,5-c]pyridin und 1,17 g K-tert.-butylat in 20 ml DMF wird unter Rühren bei 20° tropfenweise mit einer Lösung von 0,79 g Chloracetonitril in 5 ml DMF versetzt. Man rührt noch 30 Min. bei 20°, gießt auf Eis, gibt Salzsäure bis pH 6 hinzu und arbeitet wie üblich auf Man erhält 2-Butyl-3-(2'-cyan-biphenylyl-4-methyl)-5-cyanmethyl-4,5-dihydro-4-oxo-3H-imidazo[4,5-c]pyridin, F. 64°.

Analog erhält man die folgenden 2-Butyl-3-(2'-cyan-biphenylyl-4-methyl)-4,5-dihydro-4-oxo-3H-imidazo[4,5-c]pyridine:
- mit Methyliodid:: 5-Methyl-, F. 107°
- mit Ethyliodid:: 5-Ethyl-
- mit Isopropyliodid:: 5-Isopropyl-
- mit Butylbromid:: 5-Butyl-
- mit tert.-Butylbromid:: 5-tert.-Butyl-
- mit 2,2,2-Trifluorethyliodid:: 5-(2,2,2-Trifluorethyl-)-, Öl, Rf 0,3 (Ethylacetat/Hexan 9 : 1)
- mit Pentafluorethyliodid:: 5-Pentafluorethyl-
- mit 3,3,3-Trifluorpropyliodid:: 5-(3,3,3-Trifluorpropyl)-
- mit 3-Brompropionnitril:: 5-(2-Cyanethyl)-
- mit 4-Brombutyronitril:: 5-(3-Cyanpropyl)-
- mit Bromessigsäuremethylester:: 5-Methoxycarbonylmethyl-, Monohydrat, F. 82°
- mit 3-Brompropionsäureethylester:: 5-(2-Ethoxycarbonyl-ethyl)-,
- mit Allylbromid:: 5-Allyl-
- mit Propargylbromid:: 5-Propargyl-
- mit Benzylbromid:: 5-Benzyl-, F. 119°
- mit o-Fluorbenzylbromid:: 5-(o-Fluorbenzyl)-, ölig, Rf 0,56 (Ethylacetat/Hexan 1:1)
- mit m-Fluorbenzylbromid:: 5-(m-Fluorbenzyl)-, ölig, Rf 0,54 (Ethylacetat/Hexan 1:1)
- mit p-Fluorbenzylbromid:: 5-(Fluorbenzyl)-, F. 156°
- mit o-Chlorbenzylbromid:: 5-(o-Fluorbenzyl)-, F. 130°
- mit m-Chlorbenzylbromid:: 5-(m-Chlorbenzyl)-, F. 127°
- mit p-Chlorbenzylbromid:: 5-(p-Chlorbenzyl)-, F. 124°
- mit o-Brombenzylbromid:: 5-(o-Brombenzyl)-, F. 142°
- mit m-Brombenzylbromid:: 5-(m-Brombenzyl)-
- mit p-Brombenzylbromid:: 5-(p-Brombenzyl)-, F. 98°
- mit p-Methylbenzylbromid:: 5-(p-Methylbenzyl)-
- mit o-Trifluormethylbenzylbromid:: 5-(o-Trifluormethylbenzyl)-, F. 105°
- mit m-Trifluormethylbenzylbromid:: 5-(m-Trifluormethylbenzyl)-
- mit p-Trifluormethylbenzylbromid:: 5-(p-Trifluormethylbenzyl)-
- mit o-Methoxycarbonylbenzylbromid:: 5-(o-Methoxycarbonylbenzyl)-, F. 59°
- mit m-Methoxycarbonylbenzylbromid:: 5-(m-Methoxycarbonylbenzyl)-
- mit p-Methoxycarbonylbenzylbromid:: 5-(p-Methoxycarbonylbenzyl)-, F. 120°
- mit o-Ethoxycarbonylbenzylbromid:: 5-(o-Ethoxycarbonyl-benzyl)-
- mit m-Ethoxycarbonylbenzylbromid:: 5-(m-Ethoxycarbonyl-benzyl)-
- mit p-Ethoxycarbonylbenzylbromid:: 5-(p-Ethoxycarbonyl-benzyl)-
- mit o-Cyanbenzylbromid:: 5-(o-Cyanbenzyl)-, F. 65°
- mit m-Cyanbenzylbromid:: 5-(m-Cyanbenzyl)-, F. 140°
- mit p-Cyanbenzylbromid:: 5-(p-Cyanbenzyl)-
- mit o-Nitrobenzylchlorid:: 5-(o-Nitrobenzyl)-, F. 149°
- mit m-Nitrobenzylchlorid:: 5-(m-Nitrobenzyl)-
- mit p-Nitrobenzylchlorid:: 5-(p-Nitrobenzyl)-, F. 142°
- mit o-Trifluoracetamidobenzylbromid:: 5-(o-Trifluoracetamidobenzyl)-
- mit m-Trifluoracetamidobenzylbromid:: 5-(m-Trifluoracetamidobenzyl)-
- mit p-Trifluoracetamidobenzylbromid:: 5-(p-Trifluoracetamidobenzyl)-
- mit 2,6-Dichlorbenzylbromid:: 5-(2,6-Dichlorbenzyl)-, F. 178°
- mit 2-Fluor-6-nitro-benzylbromid:: 5-(2-Fluor-6-nitro-benzyl)-, F. 193°
- mit 2-Chlor-6-nitro-benzyl-bromid:: 5-(2-Chlor-6-nitro-benzyl)-, F. 206.

### Beispiel 7

Ein Gemisch von 800 mg 2-Butyl-3-(2'-cyan-biphenylyl-4-methyl)-4,5-dihydro-4-oxo-3H-imidazo[4,5-c]pyridin, 515 mg Trimethylzinnazid und 20 ml Toluol wird 96 Std. gekocht und eingedampft. Chromatographie des Rückstands (Kieselgel; Dichlormethan/Methanol 9:1, dann 85:15 und 80:20) liefert 2-Butyl-4,5-dihydro-4-oxo-3-[2'-(5-tetrazolyl)-biphenylyl-4-methyl]-3H-imidazo[4,5-c]pyridin, F. 167°. In üblicher Weise wird daraus das entsprechende K-Salz hergestellt.

Analog erhält man aus den entsprechenden Cyanverbindungen:
2-Butyl-4,5-dihydro-4-oxo-1-[2'-(5-tetrazolyl)-biphenylyl-4-methyl]-1H-imidazo[4,5-c]pyridin
2-Butyl-4,5-dihydro-4-oxo-5-[2'-(5-tetrazolyl)-biphenylyl-4-methyl]-1(oder 3)H-imidazo[4,5-c]pyridin
2-Butyl-4,5-dihydro-4-oxo-1,5-bis-[2'-(5-tetrazolyl)-biphenylyl-4-methyl]-1H-imidazo[4,5-c]pyridin
2-Butyl-4,5-dihydro-4-oxo-3,5-bis-[2'-(5-tetrazolyl)-biphenylyl-4-methyl]-3H-imidazo[4,5-c]pyridin, Tetradekahydrat; F. > 300°
2-Butyl-4,5-dihydro-4-oxo-3-[4-o-(5-tetrazolyl)-phenoxy-methyl-benzyl]-3H-imidazo[4,5-c]pyridin
2-Butyl-4,5-dihydro-4-oxo-3-[4-o-(5-tetrazolyl)-benzyloxy-benzyl]-3H-imidazo[4,5-c]pyridin.

Aus den in Beispiel 6 beschriebenen Verbindungen erhält man analog die nachstehenden 2-Butyl-4,5-dihydro-4-oxo-3-[2'-(5-tetrazolyl)-biphenylyl-4-methyl]-3H-imidazo[4,5-c]pyridine:
5-(5-Tetrazolyl)-methyl-, F. > 300°; Rf 0,07 (Ethylacetat/Methanol 1:1)
5-Methyl-
5-Ethyl-
5-Isopropyl-
5-Butyl-
5-tert.-Butyl-
5-Pentafluorethyl-
5-(3,3,3-Trifluorpropyl)-
5-[2-(5-Tetrazolyl)-ethyl]-
5[3-(5-Tetrazolyl)-propyl]-
5-Methoxycarbonylmethyl-
5-(2-Ethoxycarbonyl-ethyl)-
5-Allyl-
5-Propargyl-
5-Benzyl-, Heptahydrat, F. 130°; K-Salz, Dihydrat, F. 250°
5-(o-Fluorbenzyl)-, F. 118°; K-Salz, Tetrahydrat, F. > 300°
5-(m-Fluorbenzyl)-, F. 182° (Zers.)
5-(p-Fluorbenzyl)-, Dihydrat, F. 135°
5-(o-Chlorbenzyl)-, F. 123°; K-Salz, Dihydrat, F. 190°
5-(m-Chlorbenzyl)-, F. 126°
5-(p-Chlorbenzyl)-, Monohydrat, F. 145°
5-(o-Brombenzyl)-, F. 173°
5-(m-Brombenzyl)-
5-(p-Brombenzyl)-
5-(p-Methylbenzyl)-
5-(o-Trifluormethyl-benzyl)-
5-(m-Trifluormethyl-benzyl)-
5-(p-Trifluormethyl-benzyl)-
5-(o-Methoxycarbonyl-benzyl)-, F. 124°; K-Salz, Semihydrat, F. 152°
5-(m-Methoxycarbonyl-benzyl)-
5-(p-Methoxycarbonyl-benzyl)-, F. 188°
5-(o-Ethoxycarbonyl-benzyl)-, K-Salz, Dihydrat, F. > 300°
5-(m-Ethoxycarbonyl-benzyl)-
5-(p-Ethoxycarbonyl-benzyl)-
5-[o-(5-Tetrazolyl)-benzyl]-, F. 243°
5-[m-(5-Tetrazolyl)-benzyl]-, F. > 300°
5-[p-(5-Tetrazolyl)-benzyl]-
5-(o-Nitrobenzyl)-, F. 189°
5-(m-Nitrobenzyl)-
5-(p-Nitrobenzyl)-, F. > 300°
5-(o-Trifluoracetamido-benzyl)-
5-(m-Trifluoracetamido-benzyl)-
5-(p-Trifluoracetamido-benzyl)-
5-(2-Fluor-6-nitro-benzyl)-, F. 198°
5-(2-Chlor-6-nitro-benzyl)-, F. 144°; K-Salz, Dihydrat, F. 249°

### Beispiel 8

a) Analog Beispiel 2 erhält man aus IIIa und 4-Brommethyl-2'-(1(oder 2)-triphenylmethyl-5-tetrazolyl)-biphenyl das 2-Butyl-3-[2'-(1(oder 2) - triphenylmethyl-5-tetrazolyl-biphenylyl-4-methyl]-4,5-dihydro-4-oxo-3H-imidazol[4,5-c]pyridin, F. 127°.
b) Analog Beispiel 6 erhält man daraus mit Benzylbromid das 5-Benzyl-2-butyl-3-[2'-(1(oder 2)-triphenylmethyl-5-tetrazolyl-biphenylyl-4-methyl]-4,5-dihydro-4-oxo-3H-imidazo[4,5-c]-pyridin, F. 81°.
c) Eine Lösung von 1 g des nach b) erhaltenen Produkts in 4 ml Dichlormethan und 4 ml Methanol wird mit 4 ml etherischer HCl-Lösung versetzt und 3 Std. bei 20° gerührt Man dampft ein, arbeitet wie üblich auf und erhält nach chromatographischer Abtrennung des gebildeten Triphenylcarbinols 5-Benzyl-2-butyl-4,5-dihydro-4-oxo-3-[2'-(5-tetrazolyl)-biphenylyl-4-methyl]-3H-imidazo[4,5-c]pyridin, Heptahydrat, F. 130°. K-Salz, Dihydrat, F. 250°.

### Beispiel 9

Eine Lösung von 573 mg 2-Butyl-4,5-dihydro-5-p-methoxycarbonylbenzyl-4-oxo-3-[2'-(5-tetrazolyl)-biphenylyl-4-methyl]-3H-imidazo[4,5-c]pyridin in 17 ml THF und 6 ml Methanol wird mit 3 ml 1 n wässeriger NaOH-Lösung versetzt und 5 Std. bei 20° gerührt. Man säuert mit HCl an, arbeitet wie üblich auf und erhält 2-Butyl-5-p-carboxybenzyl-4,5-dihydro-4-oxo-3-[2'-(5-tetrazolyl)-biphenylyl-4-methyl]-3H-imidazo[4,5-c]pyridin, F. > 300°; Rf 0,26 (Ethylacetat/Methanol 1:1).

Analog erhält man durch Verseifung der entsprechenden Methylester die nachstehenden 2-Butyl-4,5-dihydro-4-oxo-3-[2'-(5-tetrazolyl)-biphenylyl-4-methyl]-3H-imidazo[4,5-c]pyridine:
- 5-o-Carboxybenzyl-,: F. 211°; Mono-K-Salz, F. > 300°, Di-K-Salz, Monohydrat, F. 283°
- 5-m-Carboxybenzyl-.:

Die nachstehenden Beispiel betreffen pharmazeutische Zubereitungen, die Wirkstoffe der Formel I oder ihre Salze enthalten.

### Beispiel A: Tabletten und Dragees

In üblicher Weise werden Tabletten folgender Zusammensetzung gepreßt, die bei Bedarf mit einer üblichen Drageedecke auf Sucrosegrundlage überzogen werden:

| | |
|---|---|
| 2-Butyl-3-(2'-cyan-biphenylyl-4-methyl]-4,5-dihydro4-oxo-3H-imidazo[4,5-c]pyridin | 100 mg |
| Mikrokristalline Cellulose | 278,8 mg |
| Lactose | 110 mg |
| Maisstärke | 11 mg |
| Magnesiumstearat | 5 mg |
| Feinteiliges Siliciumdioxid | 0,2 mg |

### Beispiel B: Hartgelatine-Kapseln

Übliche zweiteilige Hartgelatine-Kapseln werden jeweils gefüllt mit

| | |
|---|---|
| Wirkstoff der Formel I | 100 mg |
| Lactose | 150 mg |
| Cellulose | 50 mg |
| Magnesiumstearat | 6 mg |

### Beispiel C: Weichgelatine-Kapseln

Übliche Weichgetatine-Kapseln werden mit einem Gemisch aus jeweils 40 mg Wirkstoff und 250 mg Olivenöl gefüllt.

### Beispiel D: Ampullen

Eine Lösung von 200 g Wirkstoff in 2 kg 1,2-Propandiol wird mit Wasser auf 10 l aufgefüllt und in Ampullen gefüllt, so daß jede Ampulle 20 mg Wirkstoff enthält.

## Patentansprüche

1. Imidazopyndinderivate der Formel I 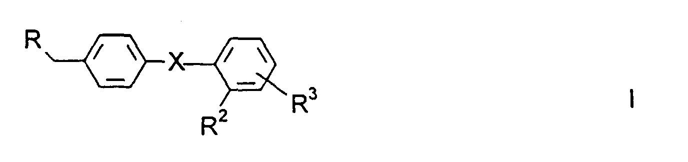 worin
R 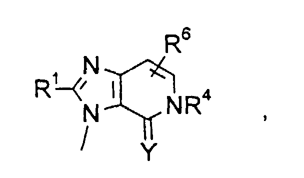
R¹ A mit bis zu 6 C-Atomen,
R² COOH, COOA, CN oder 5-Tetrazolyl,
R³ H,
R⁴ H, 5-Tetrazolyl-alkyl mit 1-6 C-Atomen mit "alkyl"-Teil, unsubstituiertes oder einfach durch Hal, COOH, COOA, CN, NO₂ oder 5-Tetrazolyl substituiertes Aralkyl mit 7-11 C-Atomen oder 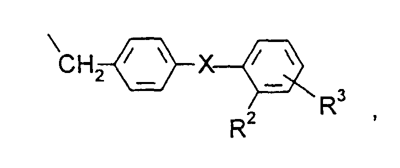
R⁶ H,
X fehlt,
Y O,
A Alkyl mit 1-6 C-Atomen und
Hal F, Cl, Br oder I bedeuten,
sowie ihre Salze.

2. 5-Benzyl-2-butyl-4,5-dihydro-4-oxo-3-[2'-(5-tetrazolyl-biphenylyl-4-methyl]-3H-imidazo[4,5-c]-pyridin und dessen Kaliumsalz.

3. Verfahren zur Herstellung von Imidazopyridinderivaten der Formel I nach Anspruch 1 sowie ihrer Salze, dadurch gekennzeichnet, daß man
(a) eine Verbindung der Formel II 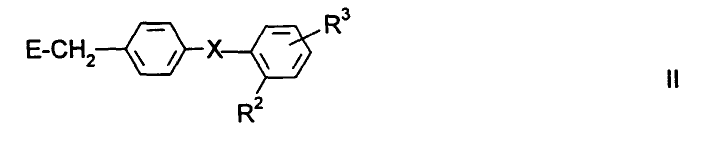 worin
E Cl, Br, I oder eine freie oder reaktionsfähig funktionell abgewandelte OH-Gruppe bedeutet und
R², R³, und X die in Anspruch 1 angegebene Bedeutung haben,
mit einer Verbindung der Formel III
H-R III
worin
R die in Anspruch 1 angegebene Bedeutung hat,
oder
(b) zur Herstellung einer Verbindung der Formel I, worin R⁴ H und Y O bedeuten, eine Verbindung der Formel IV 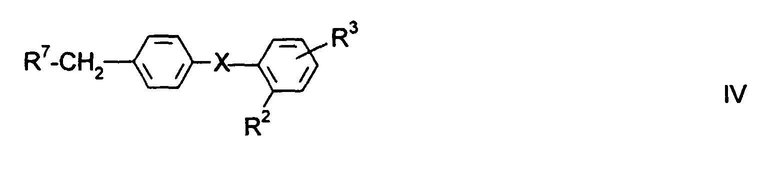 worin
R⁷ 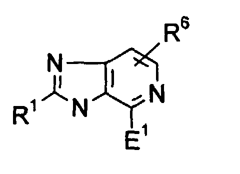 und
E¹ Cl, Br, I oder eine reaktionsfähig funktionell abgewandelte OH-Gruppe
bedeuten und
R¹, R², R³, R⁶ und X die in Anspruch 1 angegebenen Bedeutungen haben,
mit einem solvolysierenden Mittel behandelt,
oder
(c) eine Verbindung der Formel V 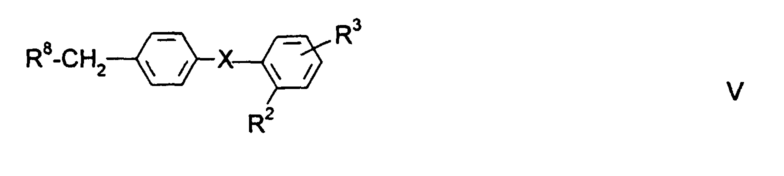 worin
R⁸ 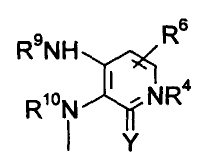
R⁹ R¹-CO oder H und
R¹⁰ H (falls R⁹ R¹-CO ist) oder R¹-CO (falls R⁹ H ist)
bedeuten
und
R¹, R², R³, R⁴, R⁶, X und Y die in Anspruch 1 angegebenen Bedeutungen haben,
mit einem cyclisierenden Mittel behandelt,
oder
(d) eine Verbindung der Formel I aus einem ihrer funktionellen Derivate durch Behandeln mit einem solvolysierenden oder hydrogenolysierenden Mittel in Freiheit setzt,
und/oder daß man in einer Verbindung der Formel I einen oder mehrere Rest(e) R und/oder R² in einen oder mehrere andere Reste R und/oder R² umwandelt und/oder eine Base oder Säure der Formel I in eines ihrer Salze umwandelt.

4. Verfahren zur Herstellung pharmazeutischer Zubereitungen, dadurch gekennzeichnet, daß man eine Verbindung der Formel I nach Anspruch 1 und/oder eines ihrer physiologisch unbedenklichen Säureadditionssalze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I nach Anspruch 1 und/oder einem ihrer physiologisch unbedenklichen Säureadditionssalze.

6. Verbindungen der Formel I nach Anspruch 1 und ihre physiologisch unbedenklichen Säureadditionssalze zur Bekämpfung von Krankheiten.

7. Verwendung von Verbindungen der Formel I nach Anspruch 1 und/oder ihrer physiologisch unbedenklichen Säureadditionssalze zur Herstellung eines Arzneimittels.

8. Verwendung von Verbindungen der Formel I nach Anspruch 1 und/oder ihrer physiologisch unbedenklichen Säureadditionssalze zur Herstellung eines Arzneimittels bei der Bekämpfung von Krankheiten.

9. Verbindungen der Formel III
H-R III
worin
R die in Anspruch 1 angegebene Bedeutung hat.

## Claims

1. An imidazopyridine derivative of formula I: 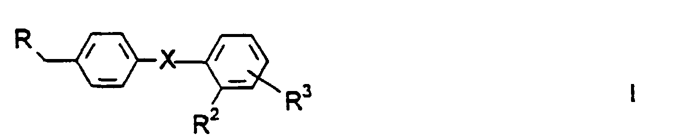 in which
R is 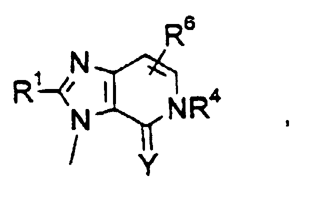
R¹ is A
R² is COOH, COOA, CN or tetrazol-5-yl,
R³ is H,
R⁴ is H, tetrazol-5-ylalkyl having 1-6 C atoms with the "alkyl" moiety, aralkyl having 7-11 C atoms which is unsubstituted or monosubstituted by Hal, COOH, COOA, CN, NO₂ or tetrazol-5-yl, or 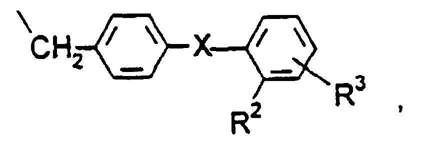
R⁶ is H,
X is absent,
Y is O,
A is alkyl having 1-6 C atoms and
Hal is F, Cl, Br or I,
and its salts.

2. 5-Benzyl-2-butyl-4,5-dihydro-4-oxo-3-[2'-(tetrazol-5-ylbiphenyl-4-ylmethyl]-3H-imidazo[4,5-c]pyridine and its potassium salt.

3. A process for the preparation of imidazopyridine derivatives of formula I according to Claim 1, and their salts, characterised in that
(a) a compound of formula II: 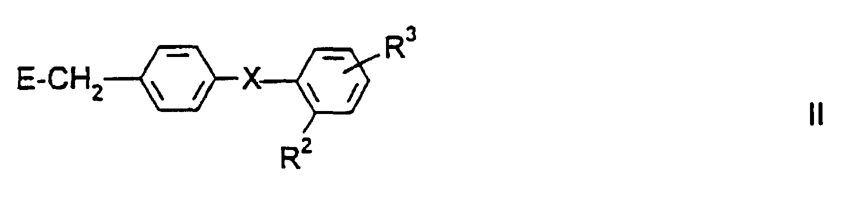 in which
E is Cl, Br, I, a free OH group or an OH group which has been functionally modified to acquire reactivity, and
R², R³ and X are as defined in Claim 1,
is treated with a compound of formula III:
H-R III
in which
R is as defined in Claim 1,
or
(b) to prepare a compound of formula I in which R⁴ is H and Y is O, a compound of formula IV: 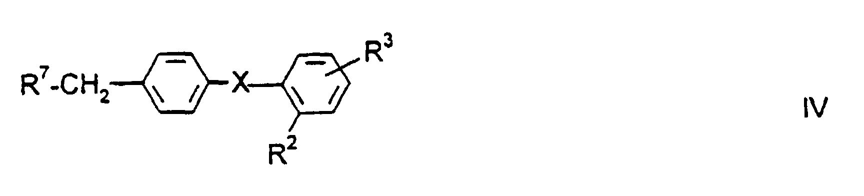 in which
R⁷ is 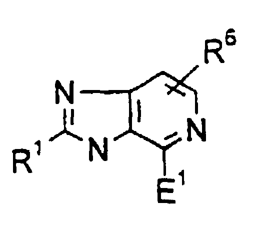 and
E¹ is Cl, Br, I or an OH group which has been functionally modified to acquire reactivity, and
R¹, R², R³, R⁶ and X are as defined in Claim 1,
is treated with a solvolysing agent, or
(c) a compound of formula V: 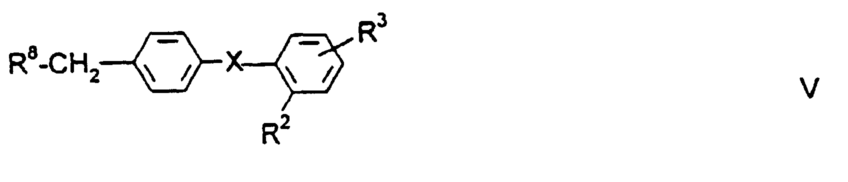 in which
R⁸ is 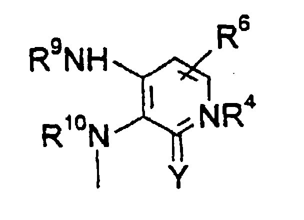
R⁹ is R¹-CO or H,
R¹⁰ is H (if R⁹ is R¹-CO) or R¹-CO (if R⁹ is H)
and
R¹, R², R³, R⁴, R⁶, X and Y are as defined in Claim 1, is treated with a cyclising agent,
or
(d) a compound of formula I is liberated from one of its functional derivatives by treatment with a solvolysing or hydrogenolysing agent,
and/or in that one or more radicals R and/or R² in a compound of formula I are converted to one or more other radicals R and/or R², and/or a base or acid of formula I is converted to one of its salts.

4. A process for the preparation of pharmaceutical formulations, characterised in that a compound of formula I according to Claim 1, and/or one of its physiologically acceptable acid addition salts, is incorporated into a suitable dosage form together with at least one solid, liquid or semiliquid excipient or adjunct.

5. A pharmaceutical formulation, characterised in that it contains at least one compound of formula I according to Claim 1, and/or one of its physiologically acceptable acid addition salts.

6. A compound of formula I according to Claim 1, and its physiologically acceptable acid addition salts, for combating diseases.

7. Use of compounds of formula I according to Claim 1, and/or their physiologically acceptable acid addition salts, for the preparation of a drug.

8. Use of compounds of formula I according to Claim 1, and/or their physiologically acceptable acid addition salts, for the preparation of a drug, in combating diseases.

9. A compound of formula III:
H-R III
in which
R is as defined in Claim 1.

## Revendications

1. Dérivés de l'imidazopyridine de formule I 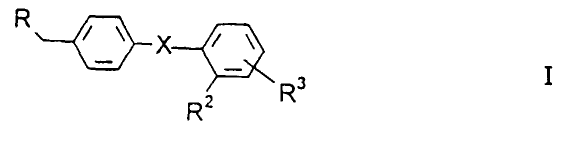
où R représente 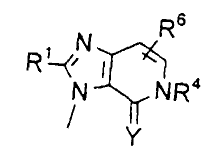
R¹ A comportant jusqu'à 6 atomes de C,
R² COOH, COOA, CN ou le 5-tétrazolyle,
R³ H,
R⁴ H, un 5-tétrazolylalkyle comportant 1 à 6 atomes de C avec la partie alkyle, un aralkyle comportant 7 à 11 atomes de C, non substitué ou monosubstitué par Hal, COOH, COOA, CN, NO₂ ou le 5-tétrazolyle ou 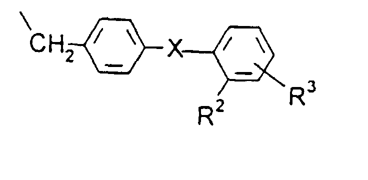
R⁶ H,
X manque,
Y O,
A un alkyle comportant 1 à 6 atomes de C et
Hal F, Cl, Br ou I,
ainsi que leurs sels.

2. La 5-benzyl-2-butyl-4,5-dihydro-4-oxo-3-[2'-(5-tétrazolylbiphénylyl)-4-méthyl]-3H-imidazo[4,5-c]pyridine et son sel de potassium.

3. Procédé pour la préparation des dérivés de l'imidazopyridine de formule I selon la revendication 1 ainsi que de leurs sels, caractérisé
(a) en ce que l'on fait réagir un composé de formule II 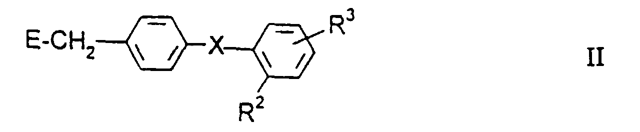 où
E représente Cl, Br, I ou un groupe OH libre ou réactif modifié fonctionnellement et
R², R³ et X ont les significations indiquées dans la revendication 1,
avec un composé de formule III :
H-R III
où R a la signification indiquée dans la revendication 1, ou
(b) en ce pour la préparation d'un composé de formule I où R⁴ représente H et Y représente O, l'on traite un composé de formule IV 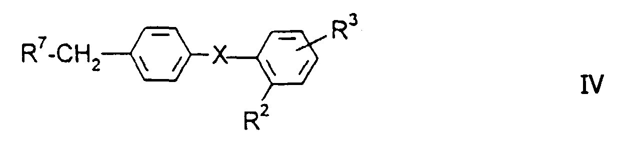
où R⁷ représente 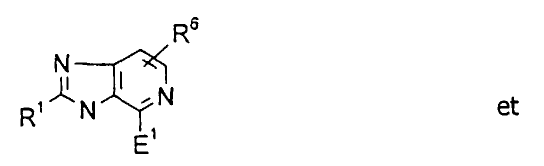
E¹ représente Cl, Br, I ou un groupe OH réactif modifié fonctionnellement, et
R¹, R², R³, R⁶ et X ont les significations indiquées dans la revendication 1, avec un agent solvolysant, ou
(c) en ce que l'on traite un composé de formule V 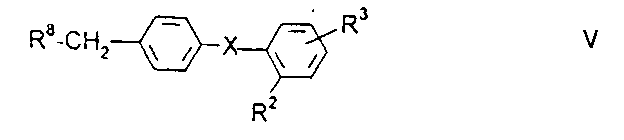
où R⁸ représente 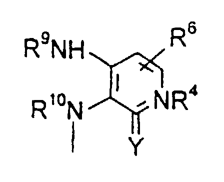
R⁹ R¹-CO ou H et
R¹⁰ H (si R⁹ est R¹-CO) ou R¹-CO (Si R⁹ est H), et
R¹, R², R³, R⁴, R⁶, X et Y ont les significations indiquées dans la revendication 1,
avec un agent de cyclisation, ou
(d) en ce que t'on libère un composé de formule I de l'un de ses dérivés fonctionnels par traitement avec un agent solvolysant ou un agent hydrogénolysant,
et/ou en ce que l'on transforme dans un composé de formule I un ou plusieurs reste(s) R et/ou R² en un ou plusieurs autre(s) reste(s) R et/ou R² et/ou une base ou un acide de formule I en l'un de ses sels.

4. Procédé pour la préparation de compositions pharmaceutiques, caractérisé en ce que l'on met un composé de formule I selon la revendication 1 et/ou l'un de ses sels d'addition d'acides physiologiquement acceptables associé à au moins un support ou un adjuvant solide, liquide ou semi-liquide sous une forme de dosage appropriée.

5. Préparation pharmaceutique caractérisée en ce qu'elle contient au moins un composé de formule I selon la revendication 1 et/ou l'un de ses sels d'addition d'acides physiologiquement acceptables.

6. Composés de formule I selon la revendication 1 et leurs sels d'addition d'acides physiologiquement acceptables pour lutter contre les maladies.

7. Utilisation des composés de formule I selon la revendication 1 et/ou de leurs sels d'addition d'acides physiologiquement acceptables pour la fabrication d'un médicament.

8. Utilisation des composés de formule I selon la revendication 1 et/ou de leurs sels d'addition d'acides physiologiquement acceptables pour la fabrication d'un médicament destiné à lutter contre les maladies.

9. Composés de formule III
H-R III
où R a la signification indiquée dans la revendication 1.
